# EUROPEAN PATENT SPECIFICATION

(11) **EP 3 701 015 B1**
(45) Date of publication and mention of the grant of the patent: **06.04.2022**
(21) Application number: 17838083.8
(22) Date of filing: 24.10.2017
(51) Int. Cl.: C12N 9/14, C12Q 1/34

(54) **AMPLIFICATION NANOSWITCH SYSTEM BASED ON SPLIT SITE-SPECIFIC CLEAVING ENZYMES FOR THE IN VITRO DETECTION OF TARGET ANALYTES AND METHOD FOR THE DETECTION OF SAID TARGET ANALYTES**
AMPLIFIKATIONSNANOSCHALTERSYSTEM AUF BASIS VON TRENNSTELLENSPEZIFISCHEN SPALTENZYMEN ZUR IN-VITRO-DETEKTION VON ZIELANALYTEN UND VERFAHREN ZUM NACHWEIS BESAGTER ZIELANALYTE
SYSTÈME DE NANOCOMMUTATEUR D'AMPLIFICATION BASÉ SUR DES ENZYMES DE CLIVAGE SPÉCIFIQUES À UN SITE FRACTIONNÉ PERMETTANT LA DÉTECTION IN VITRO D'ANALYTES CIBLES ET PROCÉDÉ PERMETTANT LA DÉTECTION DESDITS ANALYTES CIBLES

(43) Date of publication of application: 02.09.2020
(73) Proprietor: ULISSE BIOMED S.P.A., 33100 Udine (IT)
(72) Inventor: IPPODRINO, Rudy, 34135 Trieste (TS) (IT); MARINI, Bruna, 34136 Trieste (TS) (IT); MOCENIGO, Marco, 34144 Trieste (TS) (IT)
(74) Representative: Petraz, Gilberto Luigi
(86) International application number: PCT/IT2017/000233
(87) International publication number: WO 2019/082212

(56) References cited:
- WO-A1-2010/034773
- WO-A2-2018/069782
- US-A1- 2015 056 629
- WEHR MICHAEL C ET AL: "MONITORING REGULATED PROTEIN-PROTEIN INTERACTIONS USING SPLIT TEV", NATURE METHODS, NATURE PUB. GROUP, NEW YORK, vol. 3, no. 12, 29 October 2006 (2006-10-29), pages 985-993, XP009084693, ISSN: 1548-7091, DOI: 10.1038/NMETH967
- TIM SONNTAG ET AL: "An intein-cassette integration approach used for the generation of a split TEV protease activated by conditional protein splicing", MOLECULAR BIOSYSTEMS, vol. 7, no. 6, 12 April 2011 (2011-04-12), page 2031, XP055483931, GB ISSN: 1742-206X, DOI: 10.1039/c1mb05025g
- MINOU S DJANNATIAN ET AL: "Studying G protein-coupled receptor activation using split-tobacco etch virus assays", ANALYTICAL BIOCHEMISTRY, ELSEVIER, AMSTERDAM, NL, vol. 412, no. 2, 28 January 2011 (2011-01-28), pages 141-152, XP028158057, ISSN: 0003-2697, DOI: 10.1016/J.AB.2011.01.042 [retrieved on 2011-02-02]
- SUJAN S. SHEKHAWAT ET AL: "An Autoinhibited Coiled-Coil Design Strategy for Split-Protein Protease Sensors", JOURNAL OF THE AMERICAN CHEMICAL SOCIETY, vol. 131, no. 42, 28 October 2009 (2009-10-28), pages 15284-15290, XP055330102, US ISSN: 0002-7863, DOI: 10.1021/ja9050857

### SEQUENCE LISTING

<110> Ulisse Biomed S.R.L.
<120> AMPLIFICATION NANOSWITCH SYSTEM BASED ON SPLIT SITE-SPECIFIC CLEAVING ENZYMES FOR THE IN VITRO DETECTION OF TARGET ANALYTES AND METHOD FOR THE DETECTION OF SAID TARGET ANALYTES
<130> G5-1635
<160> 3
<170> BiSSAP 1.3.6
<210> 1
   <211> 117
   <212> PRT
   <213> Artificial Sequence
<220>
   <223> Modified TEV protease fragment
<400> 1
   Glu Ser Leu Phe Lys Gly Pro Arg Asp Tyr Asn Pro Ile Ser Ser Thr
   1 5 10 15
   Ile Cys His Leu Thr Asn Glu Ser Asp Gly His Thr Thr Ser Leu Tyr
   20 25 30
   Gly Ile Gly Phe Gly Pro Phe Ile Ile Thr Asn Lys His Leu Phe Arg
   35 40 45
   Arg Asn Asn Gly Thr Leu Leu Val Gln Ser Leu His Gly Val Phe Lys
   50 55 60
   Val Lys Asp Thr Thr Thr Leu Gln Gln His Leu Ile Asp Gly Arg Asp
   65 70 75 80
   Met Met Ile Ile Arg Met Pro Lys Asp Phe Pro Pro Phe Pro Gln Lys
   85 90 95
   Leu Lys Phe Arg Glu Pro Gln Arg Glu Glu Arg Ile Cys Leu Val Thr
   100 105 110
   Thr Asn Phe Gln Ala
   115
<210> 2
   <211> 124
   <212> PRT
   <213> Artificial Sequence
<220>
   <223> Modified TEV protease fragment
<400> 2
   Ser Met Ser Ser Met Val Ser Asp Thr Ser Cys Thr Phe Pro Ser Ser
   1 5 10 15
   Asp Gly Ile Phe Trp Lys His Trp Ile Gln Thr Lys Asp Gly Gln Cys
   20 25 30
   Gly Ser Pro Leu Val Ser Thr Arg Asp Gly Phe Ile Val Gly Ile His
   35 40 45
   Ser Ala Ser Asn Phe Thr Asn Thr Asn Asn Tyr Phe Thr Ser Val Pro
   50 55 60
   Lys Asn Phe Met Glu Leu Leu Thr Asn Gln Glu Ala Gln Gln Trp Val
   65 70 75 80
   Ser Gly Trp Arg Leu Asn Ala Asp Ser Val Leu Trp Gly Gly His Lys
   85 90 95
   Val Phe Met Val Lys Pro Glu Glu Pro Phe Gln Pro Val Lys Glu Ala
   100 105 110
   Thr Gln Leu Met Asn Glu Leu Val Tyr Ser Gln Gly
   115 120
<210> 3
   <211> 243
   <212> PRT
   <213> Artificial Sequence
<220>
   <223> Modified TEV protease
<400> 3
   Met Gly Glu Ser Leu Phe Lys Gly Pro Arg Asp Tyr Asn Pro Ile Ser
   1               5                   10                  15
   Ser Thr Ile Cys His Leu Thr Asn Glu Ser Asp Gly His Thr Thr Ser
               20                  25                  30
   Leu Tyr Gly Ile Gly Phe Gly Pro Phe Ile Ile Thr Asn Lys His Leu
           35                  40                  45
   Phe Arg Arg Asn Asn Gly Thr Leu Val Val Gln Ser Leu His Gly Val
       50                  55                  60
   Phe Lys Val Lys Asn Thr Thr Thr Leu Gln Gln His Leu Ile Asp Gly
   65                  70                  75                  80
   Arg Asp Met Ile Ile Ile Arg Met Pro Lys Asp Phe Pro Pro Phe Pro
                   85                  90                  95
   Gln Lys Leu Lys Phe Arg Glu Pro Gln Arg Glu Glu Arg Ile Cys Leu
               100                 105                 110
   Val Thr Thr Asn Phe Gln Thr Lys Ser Met Ser Ser Met Val Ser Asp
           115                 120                 125
   Thr Ser Cys Thr Phe Pro Ser Gly Asp Gly Ile Phe Trp Lys His Trp
       130                 135                 140
   Ile Gln Thr Lys Asp Gly Gln Cys Gly Ser Pro Leu Val Ser Thr Arg
   145                 150                 155                 160
   Asp Gly Phe Ile Val Gly Ile His Ser Ala Ser Asn Phe Thr Asn Thr
                   165                 170                 175
   Asn Asn Tyr Phe Thr Ser Val Pro Lys Asn Phe Met Glu Leu Leu Thr
               180                 185                 190
   Asn Gln Glu Ala Gln Gln Trp Val Ser Gly Trp Arg Leu Asn Ala Asp
           195                 200                 205
   Ser Val Leu Trp Gly Gly His Lys Val Phe Met Val Lys Pro Glu Glu
       210                 215                 220
   Pro Phe Gln Pro Val Lys Glu Ala Thr Gln Leu Met Asn Glu Leu Val
   225                 230                 235                 240
   Tyr Ser Gln

## Description

### FIELD OF THE INVENTION

Embodiments of the present disclosure relate to an amplification nanoswitch system based on site-specific cleaving enzymes for the *in vitro* detection of one or more target molecules in a biological sample and methods for the detection of said one or more target molecules, in particular antibodies, proteins, peptides, antigens, organic compounds, inorganic compounds, synthetic compounds and nucleic acids.

### BACKGROUND OF THE INVENTION

It is known that the detection of clinically relevant biomarkers, such as protein and nucleic acids, is essential for the diagnosis of many disease states, including infectious diseases, cancer, autoimmune diseases and allergies.

While a great variety of techniques has been studied for the direct measurement of biomarkers in whole blood, serum, saliva and other bodily fluids, many of said techniques have intrinsic limitations because they require multiple time-consuming incubation steps (Enzyme-Linked Immuno-Sorbent Assay (ELISA) and other heterogeneous, sandwich-type assays), multiple reagents, and/or sophisticated equipment.

It would be therefore desirable to develop detection methods in which molecular recognition and signal generation are integrated within a single nanoswitch system, in particular for high-throughput screening and point-of-care applications.

From a synthetic biology engineering perspective, one aspect to be considered is then how biomarker binding can be translated into a readily detectable and measurable signal change, for instance optical or electrochemical. The existing known homogeneous assays based on nanoswitch machines have proved to be less sensitive and none of them has been translated to the clinic until now. This is mainly due to the absence of an amplification system, which limits their sensitivity, and to the lack of efficiency when such assays are challenged to work directly in complex biological matrices. Known amplification systems usually are based on a "sandwich" approach, where different layers of molecules (often antibodies) increase the number of signaling molecules generated per each recognized analyte. This, however, requires labor-intensive and time-consuming incubation or washing steps.

It is further known that protein complementation (or split protein) assays are known methods exploiting the possibility to subdivide an enzyme into two catalytically inactive fragments that, when reunited (complemented), can reconstitute the enzymatic activity. This system can be used to verify protein-protein interactions and amplify the derived signal. When the two catalytically inactive fragments are conjugated to two protein factors of interest (generally named "bait" and "prey"), the complementation of the enzyme activity is enhanced if the two conjugated factors interact with each other, thus putting the two enzyme fragments in close proximity and facilitating the complementation. There is a plurality of known proteins that have been used in split protein assays, such as beta-lactamase (Remy et al., 2007), horseradish peroxidase (HRP) (Martell et al., 2016), green fluorescent proteins (GFP) (Barnard et al., 2010), beta-galactosidase (Rossi et al., 1997), dihydrofolate reductase, luciferase (Cassonnet et al., 2011), tobacco etch virus (TEV) protease (Wehr et al., 2006), and others. However, up to now protein complementation assays have been developed only for the detection of specific protein-protein interactions *in vivo*, for research studies inside living cells and not for applications such as the detection of analytes of interest in complex matrices such as whole blood.

WO 2010/034773 discloses methods of detecting analytes using split protease assays.

There is therefore a need to improve an amplification nanoswitch system based on split site-specific cleaving enzymes for the *in vitro* detection of target analytes, such as antibodies, proteins, peptides, antigens, organic compounds, inorganic compounds, synthetic compounds and nucleic acid analytes, and methods for the detection of said one or more targets, which overcome at least one of the drawbacks in the art.

Various limitations and disadvantages of conventional solutions and technologies will become apparent to one of skill in the art after reviewing the remainder of the present application with reference to the drawings and description of the embodiments which follow, though it should be understood that this description of the related art section is not intended to serve as an admission that the described subject matter is prior art.

### SUMMARY OF THE INVENTION

The present invention is set forth and characterized in the independent claims, while the dependent claims describe other characteristics of the invention or variants to the main inventive idea.

According to an aspect of the invention, an amplification nanoswitch system based on split site-specific cleaving enzymes for the *in vitro* detection of target analytes is provided. In one embodiment, said amplification nanoswitch system comprises:
- at least two catalytically inactive nanoswitch modules according to the present disclosure, each comprising:
   - at least one split catalytically inactive site-specific cleaving enzyme fragment, wherein said enzyme is a protease;
   - at least one binding moiety which is capable to bind a target analyte;
   - at least one binding arm configured to bind the at least one split catalytically inactive site-specific cleaving enzyme fragment to the at least one binding moiety;
- at least one effector molecule that contains a cleavage recognition site and a sensor or reporter molecule;
- an enhancer module configured to increase signal intensity by increasing the enzymatic activity of the amplification nanoswitch system when the binding moieties bind the target analyte, said enhancer module comprising the following specific sub-modules:
   - at least two protease catalytically inactive fragments bound to one single stranded DNA;
   - at least on linking arm molecule bound to at least one protease catalytically inactive fragment capable to do self-anneal;
   - a peptide or chemical or nucleotide spacer containing at least two protease recognition sites,
      wherein said linking arm comprises, or is constituted by RNA, peptide sequences, DNA, PNA.

According to a further aspect of the invention, a method for the *in vitro* detection of target analytes is provided. In one embodiment, said method comprises:
- providing an amplification nanoswitch system based on split site-specific cleaving enzymes as detailed above;
wherein upon interaction of one catalytically inactive nanoswitch module with the other catalytically inactive nanoswitch module induced by the presence of the target analyte, the two catalytically inactive nanoswitch modules are carried in proximity, thereby generating an enzymatic activity due to enzyme complementation, wherein the enzymatic activity leads to the cleavage of the effector molecule, wherein the presence of the cleaved effector molecule triggers emission of a detectable and measurable signal that is read or detected directly by an operator or technician and/or that is read through a reader analyzing the emitted signal whereby the result of the detection is obtained through a graphic interface connected to said reader.

In possible embodiments, combinable with all the embodiments described herein, it is provided to increase or enhance the enzymatic activity of the amplification nanoswitch system triggered when the binding moieties bind the target analyte, by using an enhancer module according to the present disclosure, thereby increasing signal intensity.

These and other features, aspects and advantages of the present disclosure will become better understood with reference to the following description, the drawings and appended claims. The drawings, which are incorporated in and constitute a part of this specification, illustrate embodiments of the present subject matter and, together with the description, serve to explain the principles of the disclosure.

### BRIEF DESCRIPTION OF THE DRAWINGS

So that the manner in which the above recited features of the present invention can be understood in detail, a more particular description of the invention, briefly summarized above, may be had by reference to embodiments. The accompanying drawings relate to embodiments of the disclosure and are described in the following:
- Figure 1a is a schematic representation of a catalytically inactive nanoswitch module;
- Figure 1b is a schematic representation of an active nanoswitch system according to embodiments described herein;
- Figure 2 is a schematic representation of the activating mechanism of the amplification nanoswitch system based on a split site-specific protease as cleaving enzyme according to embodiments described herein;
- Figure 3 shows schematic representations of different possible embodiments of the amplification nanoswitch system according to the present disclosure;
- Figure 4 is a schematic representation of different effector molecules that can be used in combination with the embodiments described herein: Panel a) Fluorescent peptide-based effector molecule. Panel b) Fluorescent nucleotide-based effector molecule. Panel c) Electrochemical peptide-based effector molecule. Panel d) Electrochemical nucleotide-based effector molecule. Panel e) Single PNA/peptide electrochemical effector molecule. Panel f) Double PNA/peptide electrochemical effector molecule;
- Figure 5 is a schematic representation of the amplification nanoswitch system with a site-specific cleaving enzyme, in presence of an enhancer module, according to embodiments described herein;
- Figure 6 is a schematic representation of the amplification system cascade for the amplification nanoswitch system with a site-specific cleaving enzyme, in presence of an enhancer module, according to embodiments described herein;
- Figure 7 is a graph showing the enhancement of the signal intensity in a TEV protease-based system comprising an enhancer module (black line; comprising A, B and C elements, later described in details), in comparison to the same system without enhancer module (dotted line; comprising only A and C elements, later described in details);
- Figure 8 is a SDS-PAGE analysis reporting activity of TEV protease enzyme, one of the possible candidate site-specific cleaving enzyme for the proposed invention, in whole blood;
- Figure 9 represents one of the possible electrochemical effector for the TEV enzyme-based system;
- Figure 10 shows the electrochemical measurement of TEV enzyme activity in whole blood following activation of the afore-mentioned electrochemical effector;
- Figure 11 depicts the decrease over time of the oxidation signal of the electrochemical reporter.

### DETAILED DESCRIPTION OF THE EMBODIMENTS

Reference will now be made in detail to the various embodiments of the invention, one or more examples of which are illustrated in the figures. Within the following description of the drawings, the same reference numbers refer to the same components. Generally, only the differences with respect to individual embodiments are described. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

Before describing these embodiments, it shall be also clarified that the present description is not limited in its application to details of the construction and disposition of the components as described in the following description using the attached drawings. The present description can provide other embodiments and can be obtained or executed in various other ways. It shall also be clarified that the phraseology and terminology used here is for the purposes of description only, and cannot be considered as limitative.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

Embodiments described herein provide amplification nanoswitch system based on split site-specific cleaving enzymes for the *in vitro* detection of target analytes and methods for the detection of said one or more targets.

It is noted here that an analytical target as used within the embodiments described herein can be at least an analytical target selected from antibodies, proteins, peptides, antigens, organic compounds, inorganic compounds, synthetic compounds and nucleic acids.

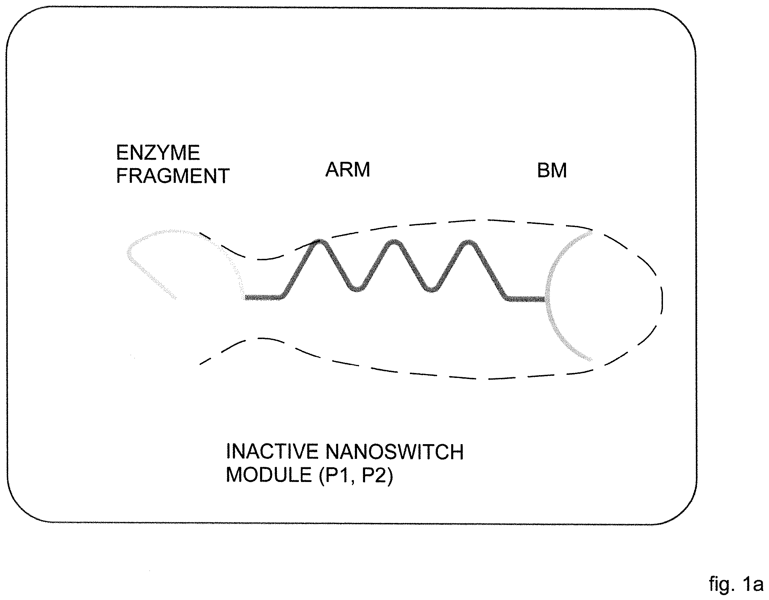

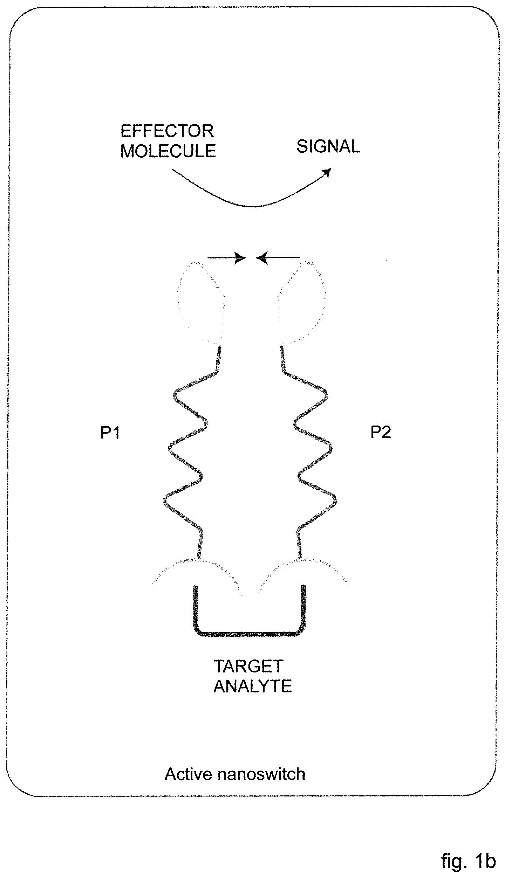

According to embodiments described using Figures 1a and 1b, the amplification nanoswitch system includes at least two catalytically inactive nanoswitch modules. A target analyte induces one catalytically inactive nanoswitch module PI to interact with the other catalytically inactive nanoswitch module P2, carrying them in close proximity, thus generating an enzymatic activity. The enzymatic activity leads to the cleavage of an effector molecule, wherein the presence of the cleaved effector molecule can emit a detectable and measurable signal that can be read or detected directly by an operator or technician (for instance in the case of colorimetric detection) and/or that is read through a reader analyzing the emitted signal whereby the result of the detection can be obtained through a graphic interface connected to said reader.

According to embodiments, the amplification nanoswitch system as described using Figure 1b includes:
- at least two catalytically inactive nanoswitch modules;
- at least one effector molecule that contains a cleavage recognition site and a sensor or reporter molecule.

A sensor or reporter molecule as used within the embodiments described herein can be a molecule emitting or generating a signal that can be detected by a reader analyzing an electric signal variation in terms of inductance, current, electric potential, in case of conductimetric, amperometric, voltammetric detection, or the presence of light at specific wavelengths in case of a colorimetric or fluorescence/chemiluminescence detection, or light scattering and/or refraction/diffraction phenomena, in case of a plasmonic optical detection.

For instance, in some implementations the sensor or reporter molecule can be a quencher (Q)/fluorophore (F) system or an electrochemical reporter (ER) or an enzyme, e.g. horseradish peroxidase (HRP), alkaline phosphate (AP), glucose oxidase (GOD), etc..., able to generate a measurable signal, or a particle, in particular a nanoparticle, or compound configured for colorimetric detection.

A catalytically inactive nanoswitch module as used in the embodiments described herein can be in turn formed by sub-modules. According to embodiments, the catalytically inactive nanoswitch module for the amplification nanoswitch system as described using Figure 1a includes the following sub-modules:
- at least one split catalytically inactive site-specific cleaving enzyme fragment, or subunit;
- at least one binding moiety (BM) which is capable to bind a target analyte;
- at least one binding arm configured to bind the at least one split catalytically inactive site-specific cleaving enzyme fragment to the at least one binding moiety (BM).

The amplification nanoswitch system based on split site-specific cleaving enzymes for the *in vitro* detection of target analytes and respective methods for the detection of said one or more targets according to embodiments described herein provide a novel and sensitive approach that allows one-step detection of target analyte, e.g. proteins, peptides, antibodies, antigens, organic compounds, inorganic compounds, synthetic compounds or nucleic acid target analytes, directly *in vitro* and in complex matrices as well using a switchable nanoswitch based on split site-specific cleaving enzyme.

An enzyme as used within the embodiments described herein can be a substance produced by a living organism that acts as a catalyst to bring about a specific biochemical reaction.

An enzyme for the enzyme fragments of the catalytically inactive nanoswitch module can be a lytic enzyme, in particular a nuclease (not covered by the claimed invention), a protease, or other lytic enzymes (not covered by the claimed invention), or a synthetic recombinant lytic enzyme (not covered by the claimed invention), that have at least 70% homology in protein sequences with bacterial, viral, eukaryotic lytic enzymes (e.g. proteases or nucleases or others) or that are chimeric (composed by fragments derived from different organisms) or synthetic.

Advantageously, the amplification nanoswitch system as described herein can be designed in a highly modular manner, thus allowing an extreme versatility.

In possible embodiments, described using Figure 1a, the enzyme as used within the embodiments described herein can be at least split into two catalytically inactive nanoswitch module PI and P2. Figure 1a is used to describe for instance catalytically inactive nanoswitch module PI, however the same Figure 1a can apply to the description of catalytically inactive nanoswitch modules P2.

Figure 1a is used to describe one catalytically inactive nanoswitch module comprising:
- at least one split catalytically inactive site-specific cleaving enzyme fragment;
- at least one binding arm bound on one side to the enzyme fragment and on the other side to a binding moiety (BM);
- at least one binding moiety (BM) which is capable to bind the target analyte.

Figure 1b is used to describe one active nanoswitch system according to embodiments of the present disclosure comprising:
- two catalytically inactive nanoswitch module each including:
   - at least two split catalytically inactive site-specific cleaving enzyme fragments, or subunits, wherein each one of said fragments, or subunits, is bound to an arm and said fragments, or subunits, are able to produce specific proteolytic cleavage when in close proximity to each other;
   - at least two binding arms wherein each one is bound to a binding moiety;
   - at least two binding moieties BM which bind the target analyte;
- at least one effector molecule that contains at least one a cleavage recognition site and contains a sensor or reporter molecule as above described.

As described using Figure 1b, in the embodiments of the present disclosure a target analyte is able to complement two complementary catalytically inactive nanoswitch modules PI and P2; the complementation allows the enzymatic fragments to act on the effector molecule generating a detectable and measurable signal which is correlated, for example proportional, to the target analyte amount.

Advantageously, the embodiments described herein define a complementation tool used for the first time for the detection of analytes *in vitro,* therefore it can be easily used as diagnostic tool or, more in general, as a tool to detect target molecules in complex matrices also in other fields of application such as agriculture, veterinary, therapy monitoring, companion diagnostics, food quality control, molecular biology research tools, etc..... Given the stability of the enzymes used in the embodiments described herein also in whole blood, together with the possibility of using specific reporter system able to work also in complex matrices, the innovative system and methods proposed in the present disclosure can work directly in blood as a no-wash, homogeneous assay, that does not require any additional incubation, lysis, washing, amplification, detection steps. The binding arms as used in the embodiments described herein are advantageous elements to modulate and obtain the correct interaction between the enzymatic fragments upon binding with the target molecule, whereas the current known complementation system and methods of the state of the art exploit recombinant chimera proteins with a rigid, immobile structure.

Importantly, in some embodiments as described hereafter, the possibility to exploit site-specific cleaving enzyme allows to induce the auto-activation of other enzyme molecules through intermediate effectors that allow the signal to be enhanced. The application of such intermediate effectors advantageously permits a no-wash, self-sustained cascade mechanism that induces signal amplification, increasing the sensitivity of the assay and the intensity of the signal, that is particularly advantageous for the system and methods described herein to be working in complex matrices.

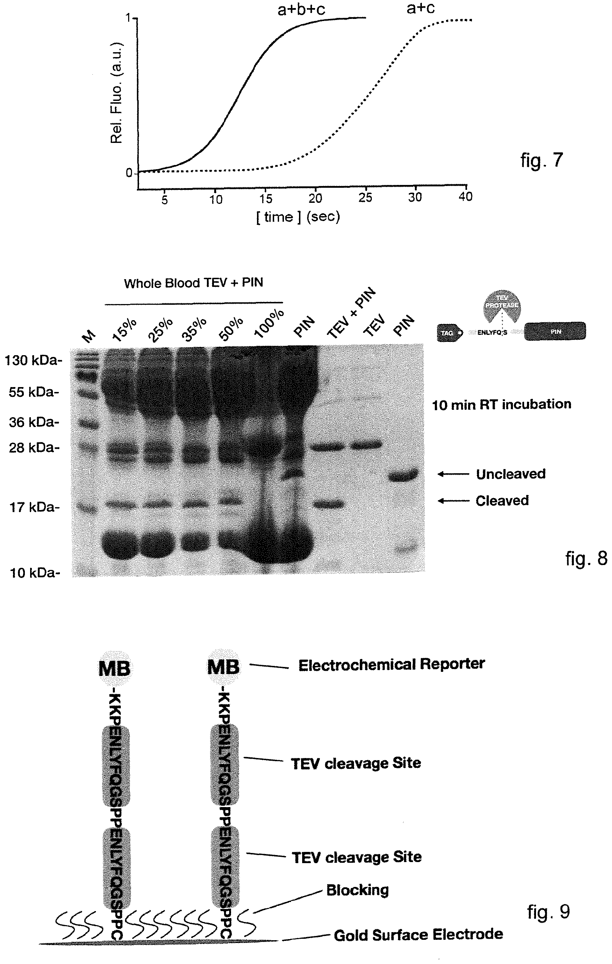

According to embodiments, which can be combined with all embodiments described herein, the amplification nanoswitch system may include an enhancer module configured to increase the signal intensity, thus ameliorating the performance in terms of sensitivity and rapidity of the assay, as it is possible to observe in Figure 7 where the comparison of the system with and without the enhancer module is presented.

### Detailed description of the amplification nanoswitch system activation mechanism

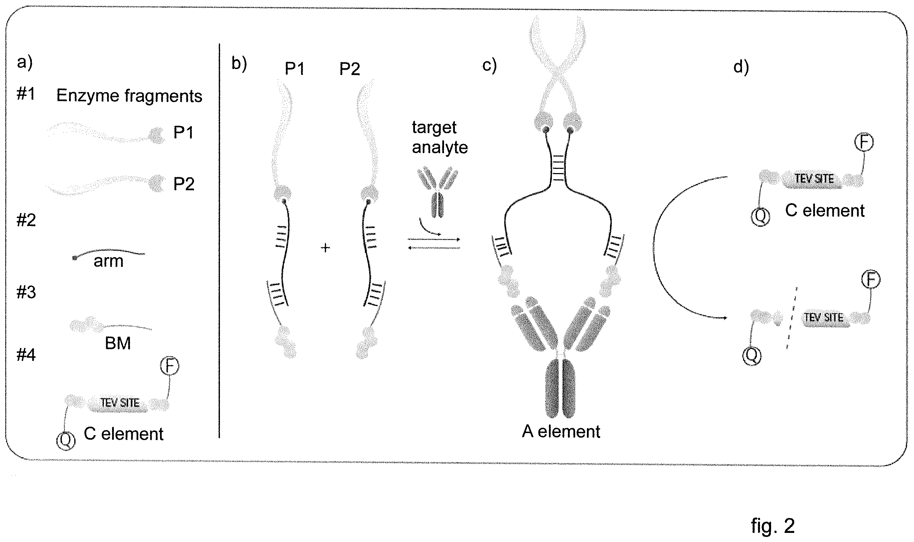

Figure 2 is used to described the activation mechanism of the amplification nanoswitch system based on a split site-specific protease as cleaving enzyme according to embodiments described herein.

For exemplification purposes, the mechanism is described using Figure 2 considering a protease as the enzyme involved (for instance a protease from Tobacco etch virus (TEV), also named TEV protease), a sensor amino acidic sequence as the cleavage recognition site in the effector molecule and a quencher/fluorophore (Q/R) system as the sensor or reporter molecule, however any other suitable enzyme and/or cleavage recognition site and/or sensor or reporter molecule could be used and the same explanation would apply.

In some embodiments, an active nanoswitch system as described above and based on a protease as site-specific cleaving enzyme comprises: at least two binding moieties BM that can bind the target molecule to be detected; at least two binding arms that facilitate the interaction of the two protease catalytically inactive fragments; at least two catalytically inactive protease fragments bound to the binding arms wherein only when the two catalytically inactive protease fragments are in close proximity, they are able to complement and generate cleavage activity of a specific target sequence; at least one effector molecule comprising at least one sensor amino acid sequence which can be cleaved from the complemented protease; at least one reporter system (for instance optical or electrochemical) that can be activated following the protease cleavage.

A possible embodiment, described using Figure 2, is explained hereby. In this case, for instance, the nanoswitch contains at least two bacterial, viral or eukaryotic catalytically inactive protease fragments that have at least 70% homology in protein sequences with bacterial, viral, eukaryotic proteases or that are chimeric (composed by fragments derived from different organisms) or synthetic (Figure 2, Panel a, #1), wherein they can generate protease activity only when they are in close proximity. Protease fragments are linked through the binding arms (Figure 2, Panel a, #2) to the binding moieties (Figure 2, Panel a, #3) that are able to recognize a target analyte. Each of the protease enzyme, binding arm and binding moiety is comprised in a respective module PI or P2 as above described.

The two protease fragments, and thus the two modules P1 and P2 do not interact in solution if the target analyte is absent (Figure 2, Panel b).

If the target analyte is present, it binds the binding moieties (BM) of two protease fragments of the two modules P1 and P2 which consequently interact with each other in close proximity, forming an active nanoswitch (Figure 2, Panel c), or A element.

Indeed, consequently to the binding of the target analyte to P1 and P2 modules, the binding arms of the modules (Figure 2, Panel a, #2) sterically facilitate the interaction of the catalytically inactive protease fragments of modules PI and P2. The close proximity of the two protease fragments of modules P1 and P2 leads to reconstitution of the protease activity. Protease activity, then, induces the cleavage of the effector molecule (C element, Figure 2, Panel a, #4) that contains a specific protease cleavage site (Figure 2, Panel d). The cleavage of the effector activates, in general, a detectable and measurable emitted signal, for instance it increases the distance between quencher (Q) and fluorophore (F) or the distance between an electrochemical reporter (ER) and electrode thereby emitting a detectable and measurable signal; the emitted signal output depends on the detection system that can be used, for instance optical or electrochemical.

### Split site-specific cleaving enzyme catalytically inactive sub-module

According to embodiments described herein, at least two site-specific catalytically inactive fragments of the modules PI and P2 can be reunited into an active enzyme if they are brought in close proximity. This complementation event can be advantageously triggered by the presence of the target analyte, and it induces the cleavage of a specific site present inside the effector molecule.

In some particular embodiments the split site-specific cleaving enzyme catalytically inactive module can be a protease.

In some particular embodiments, the aforementioned protease can be one of the following proteases: thrombin, Factor Xa, enterokinase, or the 3C proteases from Tobacco etch virus (TEV) or TEV protease or human rhinovirus, endoproteinase LysC, endoproteinase AspN, endoproteinase GluC, Furins, ubiquitin- and SUMO-specific peptidases, coxsackievirus-type picornain 3C, aarA-type peptides, lacticin 481 processing peptidase, OmpP peptidase, candidapepsins, renin, SASPase, equine infectious anaemia virus retropepsin, Mason-Pfizer leukemia virus retropepsin, murine leukemia virus type retropepsin, Rous sarcoma virus retropepsin, Tobacco vein mottling virus-type Nia peptidase, Zamp1 peptidase, Nysius proteinase, yapsin-2, cruzipain 2, CPB peptidase, cathepsin P, PibD peptidase, HIV retropepsin, bovine leukemia virus retropepsin, tropolysin, trypsin, chymotrypsin, subtilisin, proteinase K, other S8 family proteases, and others.

In some embodiments, the protease is TEV protease. TEV protease can be advantageous because, according to Applicant's experimental work, it has been proved for the first time in the art that TEV protease is catalytically active in whole blood and thus it can perform its lytic action in whole blood. This is shown in Figure 8, where a SDS-PAGE analysis of TEV activity in whole blood is reported for the first time in the art. To assess the activity of TEV protease (28kDa), different solutions containing a reporter protein, were prepared in increasing whole blood concentrations. The reporter protein used is PIN, a peptidyl-prolyl cis/trans isomerase, a recombinant protein fused to a TEV cleavage site followed by a Histidine Tag (His Tag). Wild type PIN weight is approximately 18kDa, while the recombinant uncleaved protein is around 20kDa. In the presence of TEV, there is a shift in PIN migration during electrophoresis, due to the action of the protease that is able to remove the Histidine Tag, thus shortening the protein (lane 8). The picture shows that the presence of blood does not affect TEV protease activity (lane 2 to lane 5). PIN remains uncleaved in whole blood compared to the control (lane 7 and lane 10, respectively) indicating not specific cleavage of TEV site.

The activity of TEV protease in whole blood was successively measured with an electrochemical reporter immobilized on the surface of a gold electrode (depicted in Figure 9). Briefly, a custom peptide containing two TEV cleavage sites (indicated by a rectangular shape) was synthesized and sub-sequentially modified with a Methylene Blue molecule which can be successfully measured with pulsed voltammetry assay (details in Figure 9). The peptide was attached via thiol-gold bond to working electrode surface and used as electrochemical effector (Figure 9). Deposition of a blocking molecule (mercaptohexanol, Black curved line) has been used to prevent the oxidation signal due to the whole blood presence. The N-terminal of the peptide is modified with one Methylene Blue molecule which has a specific oxidation potential. The increase/decrease of the potential value indicates the distance of the molecule from the surface and is a direct consequence of the TEV activity.

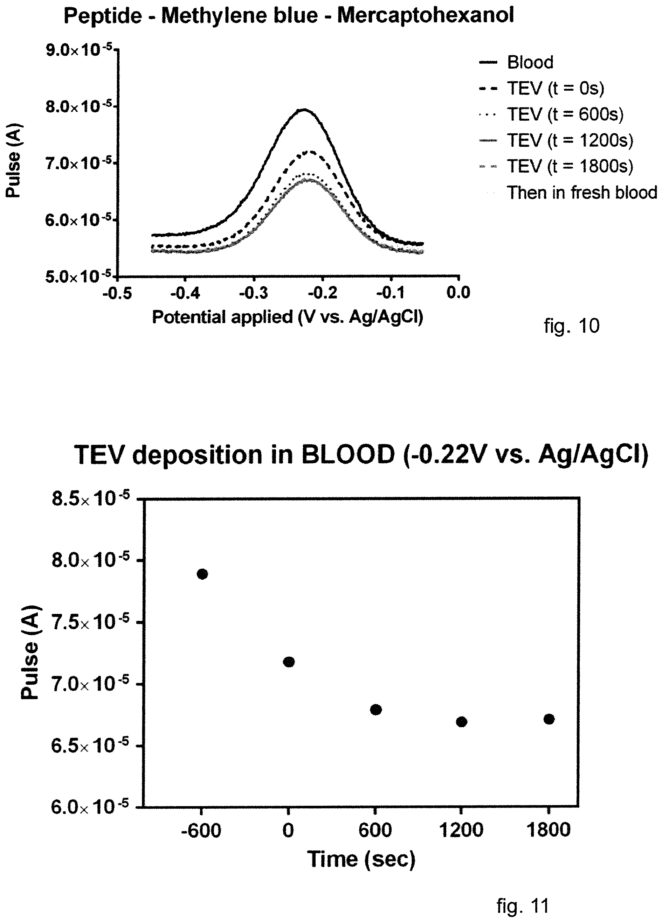

Graph shown in Figure 10 clearly demonstrates that the presence of TEV decreases the potential compared to the control (black line), indicating that electrochemical reporter detachment is occurring and this is due TEV protease cleavage activity. The intensity of the signal decreases over time and stabilizes after 10 minutes of incubation. Electrochemical variation can be measured few seconds after the addition of TEV enzyme directly in the whole blood.

The proposed system is extremely fast: one measurement takes less than 5 minutes to perform a complete analysis. The scheme shown in Figure 11 represents the decrease over time of the oxidation signal of the electrochemical reporter. It is clear how the signal reduction occurs immediately after TEV protease addition and reaches significant low value after only 10 minutes of incubation. This experiment was performed in whole blood with the addition of Heparin as anticoagulant. The oxidation peak of whole blood does not interfere with the oxidation peak of Methylene Blue (-210mV) due to its higher potential (+100mV).

In some embodiments, the TEV protease can be split into two fragments of residues 1-118 and residues 119-242 (Wehr, MC, Laage, R, Bolz, U, Fischer, TM, Grünewald, S, Scheek, S, Bach, A, Nave, KA, Rossner, MJ. (2006). Monitoring regulated protein-protein interactions using split TEV. Nature Methods. 3 (12): 985-993), albeit other pairs of protease fragments can be used according to needs, even fragments that do not naturally occur, for instance artificially modified by mutations.

In some embodiments, TEV split fragments used according to the present disclosure can derive from a synthetically engineered TEV protease enzyme, with at least 70% homology in the protein sequence with native TEV protease enzyme. For instance, the artificially modified sequence SEQ ID No. 3 as shown in the attached sequence listing is a sequence of modified TEV protease developed by the Applicant that can be used in the embodiments of the present disclosure.

In some embodiments, TEV split fragments comprise specific mutations that enhance the solubility of the two split fragments, without compromising enzymatic activity, such as Thr17Ser, Asn68Asp, Asn177Val.

In some embodiments, TEV split fragments comprise specific mutations that abolish self-cleavage, without compromising enzymatic activity, such as the site SELVYSQ (236-242) mutated to NEGGGLE, or as Ser219Val.

For example, one or more of the following fragment sequences of TEV protease developed by the Applicant can be used: SEQ ID No. 1 and SEQ ID No. 2 as shown in the attached sequence listing, which are TEV protease fragment sequences modified by the Applicant by applying specific mutations as above described and have at least 70% homology in the protein sequence with native TEV protease enzyme.

In some further embodiments not covered by the claimed invention, the split site-specific cleaving enzyme catalytically inactive module can be a nuclease.

In still further embodiments not covered by the claimed invention, the aforementioned nuclease can be a restriction enzyme cleaving a particular oligonucleotide sequence.

In yet further embodiments not covered by the claimed invention, the aforementioned nuclease can be specific for single strand target DNA.

In some embodiments not covered by the claimed invention, the aforementioned nuclease can be a mega-nuclease.

In some particular embodiments not covered by the claimed invention the split site-specific cleaving enzyme can be a glycosidase.

In some embodiments not covered by the claimed invention, instead of a lytic enzyme occurring in natural systems, the split site-specific cleaving enzyme can be a synthetic recombinant enzyme, not present in natural systems, but artificially composed by rational design exploiting different modules of existing enzymes, arranged in a functional structure, or enzymes that have at least 70% homology in protein sequences with bacterial, viral, eukaryotic lytic enzymes (e.g. proteases or nuclease) or that are chimeric (composed by fragments derived from different organisms) or synthetic.

### Binding arm sub-module

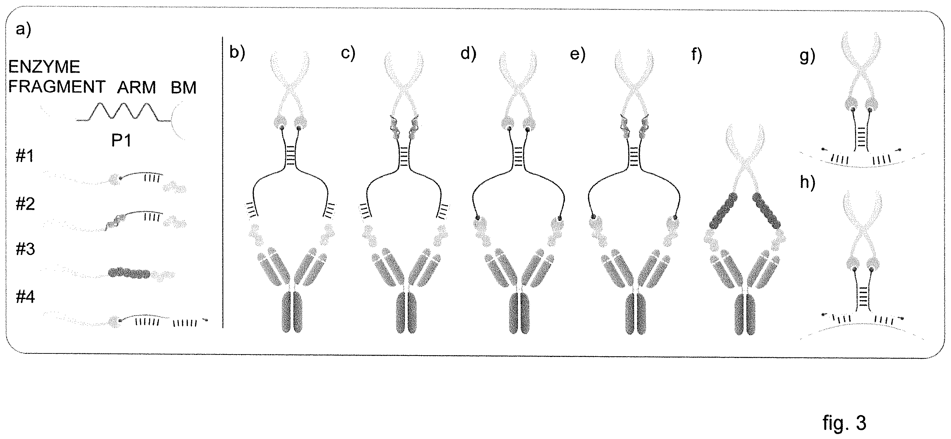

Figure 3 is used to described schemes of different possible variants of the nanoswitch based on site-specific cleaving enzyme according to the present disclosure, exploiting different combinations of binding arm and binding moieties (BM) types, for the detection of different target analytes (in Figure 3 antibodies and nucleic acids are shown only by way of example).

According to embodiments, described using Figure 3, the binding arm is the portion or sub-module that connects the binding moiety to the site-specific catalytically inactive fragment sub-module. Upon binding of the binding moieties to the targets, the binding arms facilitate the close interaction between at least two catalytically inactive fragments of the amplification nanoswitch system.

In some particular embodiments, the binding arm can be a biotinylated DNA strand (Figure 3, panel a, #1), able to bind the catalytically inactive fragment via a streptavidin or monostreptavidin unit.

In some further embodiments the binding arm can be a DNA strand (Figure 3, panel a, #2), that is able to bind the catalytically inactive fragment via a DNA sequence, e.g. as described in Shimada et al., Biotechnolgy Letters, November 2008, Volume 30, Issue 11, pp. 2001-2006, configured to bind a Hystidine tag present on the catalytically inactive fragment using nickel chelating ligand nitrilotriacetic acid (NTA).

In still further embodiments, the binding arm can be a peptide sequence fused with the enzyme fragment and with the binding moiety (Figure 3, panel a, #3), thereby creating a unique chimeric recombinant structure.

In yet further embodiments, the binding arms can be DNA strands contiguous with DNA binding moieties and biotinylated at 3' or 5' ends (Figure 3, panel a, #4).

In some particular embodiments, the binding arms can be chemically modified in order to be protected from digestion by other lytic enzymes that can be naturally present in biological samples; such protective modifications might include for instance 2'-fluorine-substituted pyrimidines, polyethylene glycol (PEG) linkage, etc.

Otherwise, for the same purpose, the binding arms can be protected from digestion by other lytic enzymes that can be naturally present in biological samples, through the usage of chemical inhibitors of such lytic enzymes.

The amplification system and methods therein therefore may include chemical compounds that can inhibit degradation of the module in whole blood.

### Binding moiety (BM) sub-module

According to embodiments, described using Figure 3, the binding moiety is able to bind the target analyte.

In some embodiments, the binding moiety can be a PNA-peptide structure, wherein the peptide can be bound by an antibody (Figure 3, panel b and c).

In some further embodiments the binding moiety can be a PNA-peptide structure, wherein the peptide can bind an antigen (not shown).

In still further embodiments the binding moiety can be a protein fused with a monostreptavidin, wherein the fusion protein can be bound by an antibody (Figure 3, panel d and e).

In yet further embodiments, the binding moiety can be an aptamer, wherein the aptamer is able to bind a target molecule; the aptamer can comprise polymers of one of the following nucleic acids: deoxyribonucleic acid (DNA), ribonucleic acid (RNA), locked nucleic acid (LNA), peptide nucleic acid (PNA), xeno nucleic acid (XNA).

In some further embodiments the binding moieties can be an antibody, wherein the antibody can bind a target molecule.

In some further embodiments, the binding moieties can be a Spiegelmer, wherein the Spiegelmer can bind a target molecule.

In still further embodiments, the binding moieties can be an antibody, wherein the antibody can bind another antibody.

In yet further embodiments, the binding moiety can be a single-chain antibody, wherein the single-chain antibody can bind a target molecule.

In some further embodiments the binding moiety can be an antibody VH region.

In some further embodiments, the binding moiety can be an affibody.

In yet further embodiments, the binding moiety can be biotinylated DNA strands (at 3' and 5') with at least a 50% homology to a nucleic acid target sequence (Figure 3, panel g).

In some further embodiments, the binding moieties can be a biotinylated RNA strand (at 3' or 5') with at least a 5%, in particular at least 10%, more in particular at least 25%, homology to a nucleic acid target sequence (Figure 3, panel h).

### Effector molecule sub-module

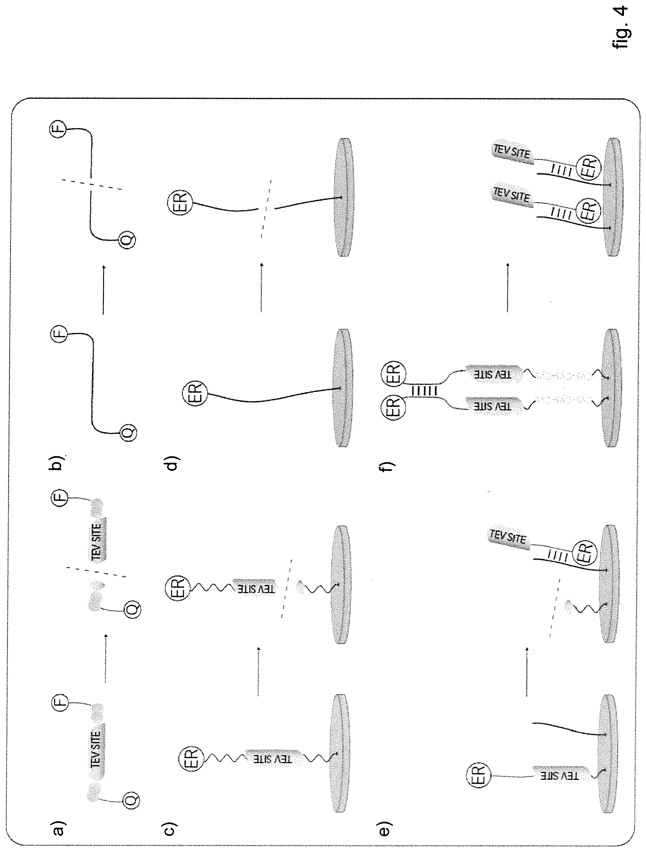

Figure 4 is used to describe possible effector molecule sub-modules as used in combination with the embodiments described herein.

According to embodiments described using Figure 4, the effector molecule is a molecule that, upon activation of the complemented active enzyme, is able to trigger a detectable signal. The nature of the effector molecule generally may depend on the catalytic activity of the chosen enzyme.

In some embodiments, the effector molecule can be a peptide sequence that can be cleaved by a complemented protease, wherein a quencher (Q) and a fluorophore (F) are conjugated at the N-terminal and at the C-terminal (or vice versa) (Figure 4, panel a). The cleavage of the sequence spatially moves away, i.e. distances, the quencher (Q) from the fluorophore (F), allowing the fluorophore to emit a fluorescence signal, proportional to the target analyte.

In some further embodiments the effector molecule can be a nucleotide sequence that can be cleaved by a complemented nuclease, wherein a quencher (Q) and a fluorophore (F) are conjugated at the 5' end and at the 3' end (or vice versa). The cleavage of the sequence spatially moves away, i.e. distances, the quencher (Q) from the fluorophore (F), allowing the fluorophore to emit a fluorescence signal (Figure 4, panel b), proportional to the target analyte.

In still further embodiments, the effector molecule can be a peptide sequence that can be cleaved by a complemented protease, wherein an electrochemical reporter (ER) is conjugated on one extremity and the other extremity is linked to a surface, that can be an electrode surface (Figure 4, panel c), that in some embodiments can be functionalized with carbon nanotube layers. The cleavage of the sequence spatially increases the distance between the electrochemical reporter (ER) and the electrode surface, triggering measurable differences in the electrochemical behavior, i.e. in the emitted electric signal, of the electrode, proportional to the target analyte.

In yet further embodiments, the effector molecule can be a nucleotide sequence that can be cleaved by a complemented nuclease, wherein an electrochemical reporter (ER) is conjugated on one extremity and the other extremity is linked to a surface, that can be an electrode surface (Figure 4, panel d). The cleavage of the sequence spatially increases the distance between the electrochemical reporter (ER) and the electrode surface, triggering measurable differences in the electrochemical behavior, i.e. in the emitted electric signal, of the electrode, proportional to the target analyte.

In further embodiments, the effector molecule can be composed by a PNA sequence coupled with a peptide sequence that can be cleaved by a complemented protease, wherein an electrochemical reporter (ER) is conjugated on the PNA extremity (Figure 4, panel e) and the peptide sequence is attached to a surface, that can be an electrode surface. The cleavage of the sequence spatially releases in the solution the PNA portion coupled with the electrochemical reporter (ER); the PNA can be designed in order to be able to hybridize with complementary DNA probes that are coated on the same electrode in a mixture with the effector molecules, or in a separate nanostructured compartment, or complementary DNA probes that are coated on a separate electrode. The hybridization between the released PNA probes with the coated DNA increases the proximity of the electrochemical reporter (ER) with the electrode surface, triggering measurable differences in the electrochemical behavior, i.e. in the emitted electric signal, of the electrode proportional to the target analyte.

In some further embodiments, the effector molecule can be composed by two PNA sequences coupled with peptide sequences that can be cleaved by a complemented protease, that are followed by cysteine-rich sequences attached via thiol bond to a surface, such as an electrode surface. The two PNA sequences, containing complementary sequences and conjugated to an electrochemical reporter (ER) on one extremity, are forced to be in close proximity by the cysteine bridges forming at the basis of the structure. The cleavage of the peptide sequences allows the PNA sequences coupled with the electrochemical reporters to be separated and released. The PNA sequences are designed in order to be able to hybridize with complementary DNA probes that are coated on the same electrode in a mixture with the effector molecules, or in a separate nanostructured compartment, or complementary DNA probes that are coated on a separate electrode. The hybridization between the released PNA probes with the coated DNA increase the proximity of the electrochemical reporter (ER) with the electrode surface, triggering measurable differences in the electrochemical behavior, i.e. in the emitted electric signal, of the electrode proportional to the target analyte.

In other embodiments, the effector molecule can be composed by different nanoparticles or beads (such as gold nanoparticles, or magnetic nanoparticles) that can be attached to each other through the sequences that can be cleaved by the split site-specific enzyme. The sequences can be attached to the nanoparticles via direct binding (thiol bonds, or EDC-NHS binding, or other chemical binding) or indirect binding by exploiting streptavidin-biotin modules. The cleavage of the sequence induces nanoparticle disaggregation and induction of a large color shift, that can be visualized by optical reader or even, in some embodiments, by naked eye by an operator or technician.

In some particular embodiments, the assay can be conducted in a non-homogenous phase, and the aforementioned nanoparticles can be attached to a surface. Following the cleavage activities, nanoparticles detach from such surface and are concentrated in a single spot or line, which renders them optically/electrochemically/colorimetric easily measurable.

In some particular embodiments, the peptide sequence can be ENLYFQG or ENLYFQS or EXXYXQ-S/G, if, for instance, the complemented protease is TEV protease.

In other embodiments, the peptide sequence can be Ala-Ala-Pro-Phe if, for instance, the complemented protease in chymotrypsin.

In some further embodiments, quencher can be one of the following molecules: BHQ1, BHQ2, BHQ3, Eclipse ^{®} Dark Quencher, TAMRA, DABCYL.

In still further embodiments, the fluorophore can be one of the following molecules: Alexa 488, Alexa680, FAM, EDANS, ATTO725, DY701, DY647, cy3, cy5, Cy5Q, Cy7Q, Iowa Black FQ, Iowa Black RQ, IRdye QC-1.

In other embodiments the fluorophore can be substituted by a luminescent molecules and the quencher can be substituted by an inhibitor of such luminescent molecule.

In further embodiments the fluorophore can be substituted by an enzyme, and the quencher can be substituted by an inhibitor of such enzyme.

In yet other particular embodiments, the electrochemical reporter (ER) can be selected from: methylene blue, ferrocene, pentamethyl ferrocene, ferrocene C-5, Nile blue, anthraquinone, anthraquinone C-5, thionine, Dabcyl, ROX, Neutral Red, gallocyanine, 2,6-dichlorophenol-indophenol.

In yet other particular embodiments, the electrochemical reporter (ER) can be an enzyme, able to trigger an electrochemical activity. For instance horseradish peroxidase (HRP), alkaline phosphatase, urease and glucose oxidase (GOD) enzyme can be used.

In still further embodiments, the electrochemical reporter (ER) can be a particle, in particular a nanoparticle, or compound configured for colorimetric detection as above described.

In some further embodiments, the effector molecule can be linked to a magnetic nanoparticle, a quantum dot nanoparticle, an agarose bead, a streptavidin-coated bead, a gold nanoparticle.

In still further embodiments, the above-mentioned electrode surface can be functionalized with carbon nanotube, fullerene, or graphene layers.

### Enhancer module

As above described, the amplification nanoswitch system comprises an enhancer module.

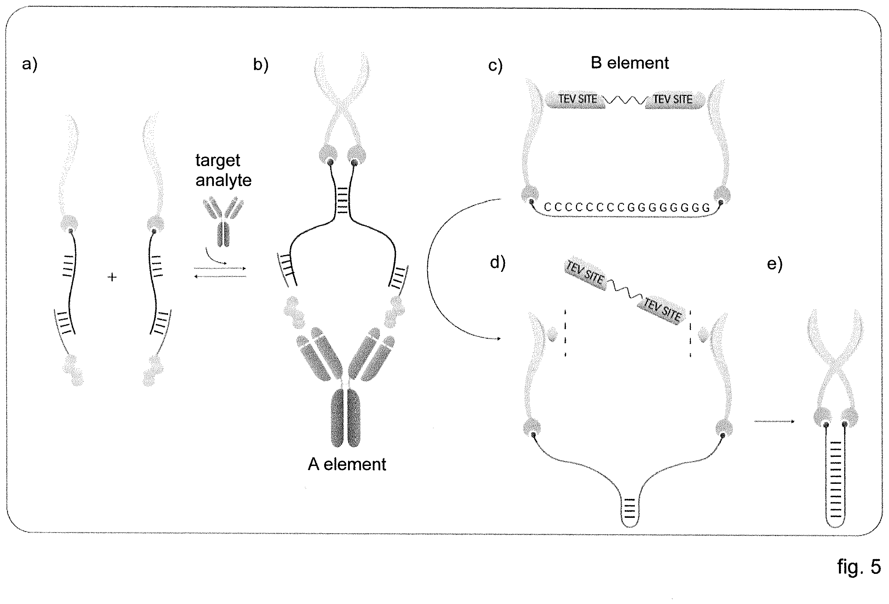

In particular, Figure 5 is used to describe an amplification nanoswitch system with site-specific cleaving enzyme based on e.g. proteases for the detection of target analytes, e.g. antibodies, in presence of an amplification molecule (enhancer module).

According to Figure 5, the enhancer (labeled as "B" element) is capable to increase or enhance the enzymatic activity of the amplification nanoswitch system triggered when the binding moieties bind the target analyte.

In Figure 5, the enhancer module comprises the following sub-modules:
- at least two site-specific cleaving enzyme catalytically inactive fragments;
- at least one linking arm;
- at least one spacer containing at least one specific cleavage site.

According to possible embodiments, the enzyme catalytically inactive fragments of the enhancer module can be fragments of enzymes which are the same, or of the same type, as used for the site-specific split enzyme of said catalytically inactive nanoswitch modules as above described.

In particular, in some embodiments the enzyme of the catalytically inactive fragments is the same as the said site-specific split enzyme of said catalytically inactive nanoswitch modules, in particular it is selected among: a nuclease (not covered by the claimed invention), a protease, or other lytic enzymes (not covered by the claimed invention), or a synthetic recombinant lytic enzyme (not covered by the claimed invention).

According to further possible embodiments not covered by the claimed invention, the enzyme catalytically inactive fragments of the enhancer module can be fragments of enzymes which are different from the enzymes used for the site-specific split enzyme of said catalytically inactive nanoswitch modules as above described. For instance, in some embodiments the enzyme catalytically inactive fragments of the enhancer module can be fragments of a luciferase, a peroxidase, a reductase, a urease or others.

In particular, in some further embodiments not covered by the claimed invention the enzyme of the catalytically inactive fragments of the enhancer module is different from the site-specific split enzyme of said catalytically inactive nanoswitch modules, in particular it is selected among: a luciferase, a peroxidase, a reductase, a urease or others.

In embodiments in which the enzyme of the catalytically inactive fragments of the enhancer module is different from the site-specific split enzyme of said catalytically inactive nanoswitch modules, the amplification nanoswitch system comprises a plurality of, in particular two or more, effector molecules, each specific for the different enzymes of the enzyme catalytically inactive fragments of the enhancer module and for the site-specific split enzyme of said catalytically inactive nanoswitch modules.

For example, in case the amplification nanoswitch system provides one enzyme for the fragments of the catalytically inactive nanoswitch modules and another different enzyme for the enzyme catalytically inactive fragments of the enhancer module, two effector molecules are provided, of which one is specific for the enzyme of the fragments of the catalytically inactive nanoswitch modules and another one is specific for the enzyme catalytically inactive fragments of the enhancer module.

According to possible specific embodiments, described using Figure 5, panel c, when the site-specific split enzyme of said catalytically inactive nanoswitch modules is a protease or a nuclease (not covered by the invention), the enhancer module contains the following specific sub-modules:
- at least two protease catalytically inactive fragments bound to one single stranded DNA, or, in alternative not covered by the claimed invention, at least two DNA/RNA nuclease catalytically inactive fragments bound to one single stranded DNA (Figure 5, panel c);
- at least on linking arm molecule bound to at least one protease, or nuclease, catalytically inactive fragment capable to do partially or completely self-anneal;
- a peptide or chemical or nucleotide spacer containing at least two protease (or nuclease or other lytic enzymes not covered by the claimed invention) recognition sites.

According to possible embodiments, combinable with all embodiments described herein, the aforementioned spacer can be a protein or a DNA or a chemical spacer.

According to possible embodiments, combinable with all embodiments described herein, the aforementioned linking arm, can comprise, or be constituted by RNA, peptide sequences, DNA, PNA.

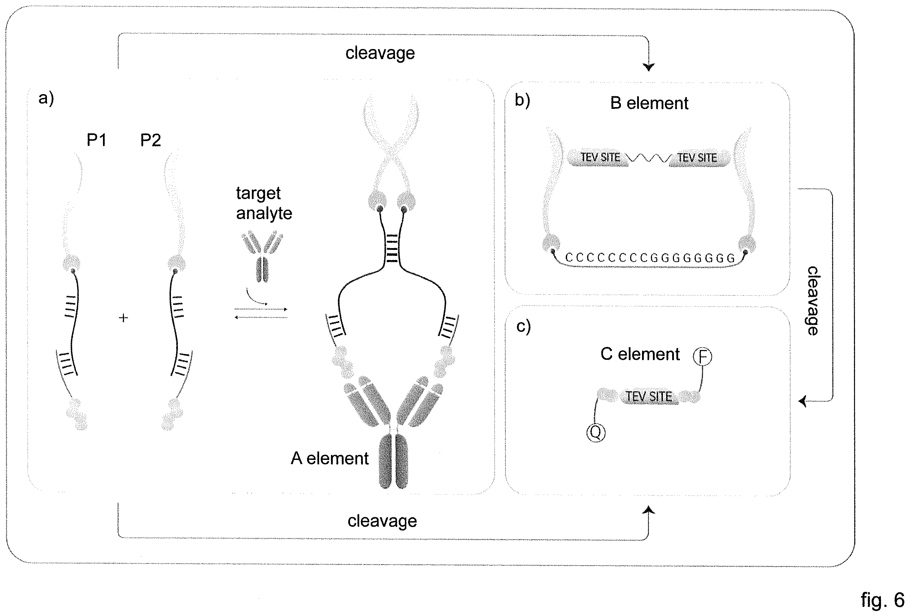

Figure 6 is used to describe the amplification system cascade for a site-specific protease-based nanoswitch for the detection of antibody according to embodiments described herein, in the presence of an enhancer module. The amplification nanoswitch system as described herein (Figure 6, panel a) is activated by the presence of the target analyte, e.g. an antibody, thereby restoring the protease activity. The protease acts both on the enhancer module (Figure 6, panel b) and directly on the effector molecule sub-modules (Figure 6, panel c shows an example of a fluorescent effector molecule). The enhancer module (Figure 6, panel b) is activated by the protease and reconstitutes new molecules of active proteases that act directly on the effector modules (Figure 6, panel c), thus amplifying the signal generated by the cleaved effector module and speeding up the reaction itself.

In embodiments using the enhancer, the amplification nanoswitch system can be activated by the presence of the target analyte, therefore restoring the enzymatic activity as above described. In such embodiments, the enzyme acts also on the enhancer module, cleaving the spacer module.

The cleavage of the spacer sub-module of the enhancer module allows the linking arm molecule to be more flexible and able to self-anneal, putting in close proximity the two enzymatic fragments (Figure 5, panel d and e), thus reconstituting the enzymatic activity. At this point, the enhancer module possesses enzymatic activity itself and is able to act on the effector sub-modules of the amplification nanoswitch system (Figure 5, panel c shows as an example of a fluorescent effector molecule). An example is reported in Figure 6, where a system based on site-specific protease nanoswitch is used to detect a target antibody. This system is able to amplify the signal generated by the increased number of cleaved effector module and speeding up the reaction itself. Indeed the first active nanoswitch (Figure 6, panel a) is able to act on two sides: both directly on the effector modules (Figure 6, panel c), and indirectly by activating other enzymes through the cleavage (and activation) of the enhancer module (Figure 6, panel b). The enhancing effect is clearly visible in Figure 7, where a graph is shown comparing the system where enhancer (B element) is present together with A and C elements (black line, a+b+c), with the system without the B element (dotted line, a+c). The presence of the enhancer boosts the reaction and shorten the time needed to reach the plateau of the signal curve

In some embodiments not covered by the claimed invention, as above described, the enhancer module can include enzyme fragments of a different enzyme (e.g. a luciferase, a peroxidase, a reductase, a urease or others) with respect to the enzymes used for the enzyme fragments of the catalytically inactive nanoswitch modules, thus triggering a different type of signal that can be read in parallel with the signal obtained by the effector module of the amplification nanoswitch system.

### Target analytes

According to embodiments, combinable with all embodiments described herein, using the amplification nanoswitch system of the present disclosure it is possible to detect a variety of molecules or target analytes. For instance, the target analyte can be chosen from: an antibody, an affibody, a single chain antibody, an antibody VH region, a peptide, a protein, a DNA strand, a RNA strand, an inorganic compound, a mono-, di- or polysaccharide, a hapten, a synthetic compound.

According to further embodiments, amplification nanoswitch systems and related methods of the present disclosure can be used in a multiplex approach detecting analysis, whereby a plurality of amplification nanoswitch systems can be used, each configured for amplification and detection of a specific and different target analyte.

While the foregoing is directed to embodiments of the invention, other and further embodiments of the invention may be devised without departing from the basic scope thereof, and the scope thereof is determined by the claims that follow.

### BIBLIOGRAPHY

Barnard, E. and Timson, DJ. (2010). Split-EGFP Screens for the Detection and Localisation of Protein-Protein Interactions in Living Yeast Cells. Molecular and Cell Biology Methods for Fungi. Methods in Molecular Biology. 638. pp. 303-317.

Cassonnet, P, Rolloy, C, Neveu, G, Vidalain, PO, Chantier, T, Pellet, J, Jones, L, Muller, M, Demeret, C, Gaud, G, Vuillier, FO, Lotteau, V, Tangy, FD, Favre, M, Jacob, Y. (2011). Benchmarking a luciferase complementation assay for detecting protein complexes. Nature Methods. 8 (12): 990-992.

Martell JD, Yamagata M, Deerinck TJ, Phan S, Kwa CG, Ellisman MH, Sanes JR, Ting AY. (2016) A split horseradish peroxidase for the detection of intercellular protein-protein interactions and sensitive visualization of synapses. Nat Biotechnol. 2016 Jul;34(7):774-80.

Remy, I, Ghaddar, G, Michnick, SW. (2007) Using the β-lactamase protein-fragment complementation assay to probe dynamic protein-protein interactions. Nature Protocols. 2 (9): 2302-2306.

Rossi, F, Charlton, CA, Blau, HM. (1997). Monitoring protein-protein interactions in intact eukaryotic cells by beta-galactosidase complementation. Proceedings of the National Academy of Sciences of the United States of America. 94 (16): 8405-8410.

Wehr, MC, Laage, R, Bolz, U, Fischer, TM, Grünewald, S, Scheek, S, Bach, A, Nave, KA, Rossner, MJ. (2006). Monitoring regulated protein-protein interactions using split TEV. Nature Methods. 3 (12): 985-993.

## Claims

1. An amplification nanoswitch system based on split site-specific cleaving enzymes for the *in vitro* detection of target analytes, said amplification nanoswitch system comprising:
- at least two catalytically inactive nanoswitch modules (P1, P2) each comprising:
- at least one split catalytically inactive site-specific cleaving enzyme fragment, wherein said enzyme is a protease;
- at least one binding moiety (BM) which is capable to bind a target analyte;
- at least one binding arm configured to bind the at least one split catalytically inactive site-specific cleaving enzyme fragment to the at least one binding moiety (BM);
- at least one effector molecule that contains a cleavage recognition site and a sensor or reporter molecule;
- an enhancer module configured to increase signal intensity by increasing the enzymatic activity of the amplification nanoswitch system when the binding moieties bind the target analyte, said enhancer module comprising the following specific sub-modules:
- at least two protease catalytically inactive fragments bound to one single stranded DNA;
- at least on linking arm molecule bound to at least one protease catalytically inactive fragment capable to do self-anneal;
- a peptide or chemical or nucleotide spacer containing at least two protease recognition sites.
wherein said linking arm comprises, or is constituted by RNA, peptide sequences, DNA, PNA.

2. The amplification nanoswitch system of claim 1, wherein upon interaction of one catalytically inactive nanoswitch module PI with the other catalytically inactive nanoswitch module (P2) induced by the presence of the target analyte, the two catalytically inactive nanoswitch modules (P1, P2) are able to be carried in proximity, thereby generating an enzymatic activity due to enzyme complementation, wherein the enzymatic activity leads to the cleavage of the effector molecule, wherein the presence of the cleaved effector molecule triggers emission of a detectable and measurable signal that is read or detected directly by an operator or technician and/or that is read through a reader analyzing the emitted signal whereby the result of the detection is obtained through a graphic interface connected to said reader.

3. The amplification nanoswitch system of claim 1 or 2, wherein said sensor or reporter molecule is chosen among:
- a molecule emitting a signal that can be detected by a reader and/or a user analyzing an electric signal variation in terms of inductance, current, electric potential, in case of conductimetric, amperometric, voltammetric detection, or the presence of light at specific wavelengths in case of a colorimetric or fluorescence/chemiluminescence detection, or light scattering and/or refraction/diffraction phenomena, in case of a plasmonic optical detection, or a combination thereof, or
- a quencher/fluorophore system or an electrochemical reporter, or an enzyme able to generate a measurable signal, or a particle or compound configured for colorimetric detection, in particular nanoparticles or beads that can be attached to each other through sequences that can be cleaved by the split site-specific enzyme, whereby the cleavage of the sequence induces nanoparticle or bead disaggregation and induction of a color shift, that can be visualized by optical reader or by an operator or technician.

4. The amplification nanoswitch system of any of claims 1 to 3, wherein said effector molecule is chosen among:
- a peptide sequence that is able to be cleaved by a complemented protease, wherein a quencher and a fluorophore are conjugated at the N-terminal and at the C-terminal, or vice versa, wherein said fluorophore may be substituted by a luminescent molecules or an enzyme and the quencher is substituted respectively an inhibitor of said luminescent molecule or an inhibitor of said enzyme or
- a peptide sequence that is able to be cleaved by a complemented protease, wherein an electrochemical reporter is conjugated on one extremity and the other extremity is linked to a surface, in particular an electrode surface or
- a peptide sequence that is able to be cleaved by a complemented protease, wherein an electrochemical reporter is conjugated on a PNA extremity and the peptide sequence is attached to a surface, in particular an electrode surface or
- said effector molecule comprises two PNA sequences coupled with peptide sequences that are able to be cleaved by a complemented protease, that are followed by cysteine-rich sequences attached via thiol bond to a surface, in particular an electrode surface.

5. The amplification nanoswitch system of claim 4, wherein said electrode surface is functionalized with carbon nanotube, fullerene, or graphene layers and said electrochemical reporter is selected from: methylene blue, ferrocene, pentamethyl ferrocene, ferrocene C-5, Nile blue, anthraquinone, anthraquinone C-5, thionine, Dabcyl, ROX, Neutral Red, gallocyanine, 2,6-dichlorophenol-indophenol.

6. The amplification nanoswitch system of any of claims 1 to 5, wherein said effector molecule is linked to any of the following: magnetic nanoparticle, or a quantum dot nanoparticle, an agarose bead, or a streptavidin-coated bead, or a combination thereof.

7. The amplification nanoswitch system of any of claims 1 to 6, wherein the analytical target is selected from: antibodies, proteins, peptides, antigens, organic compounds, inorganic compounds, synthetic compounds and nucleic acids in particular an antibody, an affibody, a single chain antibody, an antibody VH region, a peptide, a protein, a DNA strand, is a RNA strand, an inorganic compound, a mono-, di- or polysaccharide, a hapten, a synthetic compound.

8. The amplification nanoswitch system of any of claims 1 to 7, wherein said amplification nanoswitch system comprises a plurality of, in particular two or more, effector molecules, each specific for the different enzymes of the enzyme catalytically inactive fragments of the enhancer module and for the site-specific split enzyme of said catalytically inactive nanoswitch modules.

9. The amplification nanoswitch system of any of claims 1 to 8, wherein said amplification nanoswitch system comprises chemical inhibitors configured to inhibit other lytic enzymes that are naturally present in biological samples in order to protect said binding arms from digestion by said other lytic enzymes.

10. The amplification nanoswitch system of any of claims 1 to 9, wherein said protease is Tobacco etch virus (TEV) protease, in particular wherein said TEV protease is split into two fragments of residues 1-118 and residues 119-242 or wherein the TEV protease is split into the following artificially modified TEV protease fragment sequences: SEQ ID No. 1, SEQ ID No. 2, of artificially modified TEV protease having sequence SEQ ID No. 3.

11. The amplification nanoswitch system of any of claims 1 to 10, wherein said binding arm is chosen among:
- a biotinylated DNA strand able to bind the catalytically inactive fragment via a streptavidin or monostreptavidin unit;
- a DNA strand able to bind the catalytically inactive fragment via a DNA sequence configured to bind an Hystidine tag present on the catalytically inactive fragment using nickel chelating ligand nitrilotraicetic acid (NTA);
- a peptide sequence fused with the enzyme fragment and with the binding moiety, thereby creating a unique chimeric recombinant structure;
- a DNA strand contiguous with DNA binding moieties and biotinyilated at 3' or 5' ends.

12. The amplification nanoswitch system of any of claims 1 to 11, wherein said binding arm is chemically modified in order to be protected from digestion by other lytic enzymes that can be naturally present in biological samples.

13. The amplification nanoswitch system of any of claims 1 to 12, wherein said binding moiety is chosen among:
- a PNA-peptide structure, wherein the peptide is able to be bound by an antibody;
- a PNA-peptide structure, wherein the peptide is able to bind an antigen;
- a protein fused with a monostreptavidin, wherein the fusion protein is able to be bound by an antibody;
- an aptamer, wherein the aptamer is able to bind an antigen, further wherein the aptamer comprises polymers of one of the following nucleic acids: deoxyribonucleic acid (DNA), ribonucleic acid (RNA), locked nucleic acid (LNA), peptide nucleic acid (PNA), xeno nucleic acid (XNA);
- a Spiegelmer, wherein the Spiegelmer can bind a target molecule;
- an antibody, wherein said antibody is able bind an antigen or another antibody;
- a single-chain antibody, wherein the single-chain antibody is able to bind an antigen;
- an antibody VH region;
- an affibody;
- a biotinylated DNA strand with at least a 50% homology to a nucleic acid target sequence;
- a biotinylated RNA strand with at least a 5% homology to a nucleic acid target sequence.

14. A method for the *in vitro* detection of target analytes in a biological sample, said method comprising:
- providing an amplification nanoswitch system based on split site-specific cleaving enzymes according to any of claims 1 to 13;
wherein upon interaction of one catalytically inactive nanoswitch module (P1) with the other catalytically inactive nanoswitch module (P2) induced by the presence of the target analyte, the two catalytically inactive nanoswitch modules (P1, P2) are carried in proximity, thereby generating an enzymatic activity due to enzyme complementation, wherein the enzymatic activity leads to the cleavage of the effector molecule, wherein the presence of the cleaved effector molecule triggers emission of a detectable and measurable signal that is read or detected directly by an operator or technician and/or that is read through a reader analyzing the emitted signal whereby the result of the detection is obtained through a graphic interface connected to said reader said method further comprising increasing the enzymatic activity of the amplification nanoswitch system when the binding moieties bind the target analyte by using said enhancer module, thereby increasing signal intensity.

15. The method according to claim 14, wherein said sample is a whole blood sample.

## Patentansprüche

1. Amplifikations-Nanoschalter-System auf Grundlage von geteilten, ortsspezifischen Spaltungsenzymen für die In-vitro-Detektion von Target-Analyten, wobei das Amplifikations-Nanoschalter-System aufweist:
- mindestens zwei katalytisch inaktive Nanoschalter-Module (Pl, P2), die jeweils aufweisen:
- mindestens ein geteiltes, katalytisch inaktives, ortsspezifisches Spaltungs-Enzymfragment, wobei das Enzym eine Protease ist;
- mindestens einen Bindungsteil (BM), der in der Lage ist einen Target-Analyten zu binden;
- mindestens einen Bindungsarm, der geeignet ist, das mindestens eine geteilte, katalytisch inaktive, ortsspezifische Spaltungs-Enzymfragment an den mindestens einen Bindungsteil (BM) zu binden;
- mindestens ein Effektormolekül, welches eine Spaltungs-Erkennungsstelle enthält, und ein Sensor- oder Reportermolekül;
- ein Verstärkermodul, das geeignet ist, die Signalintensität zu erhöhen, indem es die enzymatische Aktivität des Amplifikations-Nanoschalter-Systems erhöht, wenn die Bindungsteile den Target-Analyten binden, wobei das Verstärkermodul die folgenden spezifischen Untermodule aufweist:
- mindestens zwei katalytisch inaktive Proteasefragmente, die an eine einzelstrang DNA gebunden sind;
- mindestens ein Verbindungsarmmolekül, welches an mindestens ein katalytisch inaktives Proteasefragment gebunden ist, das zur Selbst-Hybridisierung fähig ist;
- ein Peptid- oder Chemischer- oder Nukleotid-Spacer, welcher mindestens zwei Protease-Erkennungsstellen enthält;
wobei der Verbindungsarm RNA, Peptidsequenzen, DNA, PNA aufweist oder daraus besteht.

2. Amplifikations-Nanoschalter-System gemäß Anspruch 1, wobei bei Wechselwirkung eines katalytisch inaktiven Nanoschalter-Moduls Pl mit dem anderen katalytisch inaktiven Nanoschalter-Modul (P2), die durch die Anwesenheit des Target-Analyten induziert wird, die beiden katalytisch inaktiven Nanoschalter-Module (P1, P2) in der Lage sind in räumliche Nähe gebracht zu werden, wodurch eine enzymatische Aktivität aufgrund von Enzymkomplementierung erzeugt wird, wobei die enzymatische Aktivität zur Spaltung des Effektormoleküls führt, wobei die Anwesenheit des gespaltenen Effektormoleküls die Emission eines detektierbaren und messbaren Signals auslöst, das direkt von einem Bediener oder Techniker ausgelesen oder detektiert wird und/oder das durch ein Lesegerät ausgelesen wird, dass das emittierte Signal analysiert, wobei das Ergebnis der Detektion über eine mit dem Lesegerät verbundene, grafische Schnittstelle erhalten wird.

3. Amplifikations-Nanoschalter-System gemäß Anspruch 1 oder 2, wobei das Sensor- oder Reportermolekül ausgewählt ist, unter:
- einem Molekül das ein Signal emittiert, das von einem Lesegerät und/oder einem Benutzer detektiert werden kann, das bzw. der eine elektrische Signaländerung in Form von Induktivität, Strom, elektrischem Potenzial im Falle einer konduktometrischen, amperometrischen oder voltametrischen Detektion oder die Anwesenheit von Licht bei bestimmten Wellenlängen im Falle einer kolorimetrischen Detektion oder Fluoreszenz-/Chemilumineszenz-Detektion oder Lichtstreuung und/oder Brechungs-/Beugungsphänomene im Falle einer plasmonischen, optischen Detektion, oder eine Kombination dieser analysiert, oder
- einem Quencher-/Fluorophor-System oder einem elektrochemischen Reporters oder einem Enzym, das in der Lage ist ein messbares Signal zu erzeugen, oder einem Partikel oder einer Verbindung, die für eine kolorimetrischen Detektion geeignet ist, insbesondere Nanopartikel oder Kügelchen, die durch Sequenzen aneinander gebunden werden können, die durch das geteilte, ortsspezifische Enzym gespalten werden können, wobei die Spaltung der Sequenz die Entmischung der Nanopartikel oder Kügelchen und eine Farbverschiebung hervorruft, die durch ein optisches Lesegerät oder durch einen Bediener oder Techniker visualisiert werden kann.

4. Amplifikations-Nanoschalter-System gemäß irgendeinem der Ansprüche 1 bis 3, wobei das Effektormolekül ausgewählt wird unter:
- einer Peptidsequenz, die in der Lage ist durch eine komplementäre Protease gespalten zu werden, wobei ein Quencher und ein Fluorophor am N-Terminus und am C-Terminus, oder umgekehrt, konjugiert sind, wobei der Fluorophor durch ein lumineszierendes Molekül oder ein Enzym substituiert sein kann und der Quencher durch einen entsprechenden Inhibitor des lumineszierenden Moleküls oder einen Inhibitor des Enzyms substituiert ist oder
- einer Peptidsequenz, die in der Lage ist durch eine komplementäre Protease gespalten zu werden, wobei ein elektrochemischer Reporter an ein äußerstes Ende konjugiert ist und das andere äußerste Ende an eine Oberfläche, insbesondere eine Elektrodenoberfläche, gebunden ist, oder
- einer Peptidsequenz, die in der Lage ist durch eine komplementäre Protease gespalten zu werden, wobei ein elektrochemischer Reporter an ein äußerstes PNA-Ende konjugiert ist und die Peptidsequenz an eine Oberfläche, insbesondere eine Elektrodenoberfläche, gebunden ist oder
- wobei das Effektormolekül zwei PNA-Sequenzen aufweist, die mit Peptidsequenzen, die in der Lage sind durch eine komplementäre Protease gespalten zu werden, gekoppelt sind, und auf die cysteinreiche Sequenzen folgen, die über eine Thiolbindung an eine Oberfläche, insbesondere eine Elektrodenoberfläche, gebunden sind.

5. Amplifikations-Nanoschalter-System gemäß Anspruch 4, wobei die Elektrodenoberfläche mit Kohlenstoffnanoröhren-, Fulleren- oder Graphenschichten funktionalisiert ist und der elektrochemische Reporter ausgewählt ist aus: Methylenblau, Ferrocen, Pentamethylferrocen, Ferrocen C-5, Nilblau, Anthrachinon, Anthrachinon C-5, Thionin, Dabcyl, ROX, Neutralrot, Gallocyanin, 2,6-Dichlorphenol-Indophenol.

6. Amplifikations-Nanoschalter-System gemäß irgendeinem der Ansprüche 1 bis 5, wobei das Effektormolekül an eines der folgenden gebunden ist: ein magnetisches Nanopartikel oder ein Quantenpunkt-Nanopartikel, ein Agarosekügelchen oder ein mit Streptavidin beschichtetes Kügelchen oder eine Kombination dieser.

7. Amplifikations-Nanoschalter-System gemäß irgendeinem der Ansprüche 1 bis 6, wobei das analytische Target ausgewählt ist aus: Antikörpern, Proteinen, Peptiden, Antigenen, organischen Verbindungen, anorganischen Verbindungen, synthetischen Verbindungen und Nukleinsäuren, insbesondere einem Antikörper, einem Affikörper, einem einkettigen Antikörper, einer Antikörper-VH-Region, einem Peptid, einem Protein, einem DNA-Strang, einem RNA-Strang, einer anorganischen Verbindung, einem Mono-, Di- oder Polysaccharid, einem Hapten, einer synthetischen Verbindung.

8. Amplifikations-Nanoschalter-System gemäß irgendeinem der Ansprüche 1 bis 7, wobei das Amplifikations-Nanoschalter-System eine Mehrzahl von, insbesondere zwei oder mehr, Effektormolekülen aufweist, die jeweils spezifisch für die verschiedenen Enzyme der katalytisch inaktiven Enzymfragmente des Verstärkermoduls und für das ortsspezifische Spaltungsenzym der katalytisch inaktiven Nanoschalter-Module sind.

9. Amplifikations-Nanoschalter-System gemäß irgendeinem der Ansprüche 1 bis 8, wobei das Amplifikations-Nanoschalter-System chemische Inhibitoren aufweist, die geeignet sind, andere lytische Enzyme, die natürlicherweise in biologischen Proben vorkommen, zu hemmen, um die Bindungsarme vor dem Digerieren durch die anderen lytischen Enzyme zu schützen.

10. Amplifikations-Nanoschalter-System gemäß irgendeinem der Ansprüche 1 bis 9, wobei die Protease Tobacco-Etch-Virus (TEV)-Protease ist, insbesondere wobei die TEV-Protease in zwei Fragmente der Reste 1-118 und der Reste 119-242 geteilt ist oder wobei die TEV-Protease in die folgenden künstlich modifizierten TEV-Protease-Fragment-Sequenzen geteilt ist: SEQ ID Nr. 1, SEQ ID Nr. 2, von künstlich modifizierter TEV-Protease mit der Sequenz SEQ ID Nr. 3.

11. Amplifikations-Nanoschalter-System gemäß irgendeinem der Ansprüche 1 bis 10, wobei der Bindungsarm ausgewählt ist, unter:
- einem biotinylierten DNA-Strang, der in der Lage ist, das katalytisch inaktive Fragment über eine Streptavidin- oder Monostreptavidin-Einheit zu binden;
- einem DNA-Strang, der in der Lage ist, das katalytisch inaktive Fragment über eine DNA-Sequenz zu binden, die geeignet ist ein auf dem katalytisch inaktiven Fragment vorhandenes Hystidin-Tag, unter Verwendung des Nickel-Chelat bildenden Liganden Nitrilotriessigsäure (NTA), zu binden;
- einer Peptidsequenz, die mit dem Enzymfragment und dem Bindungsteil verbunden ist, wodurch eine spezifische chimäre, rekombinante Struktur entsteht;
- einem DNA-Strang, der an die DNA-Bindungsteile angrenzt und an den 3'- oder 5'-Enden biotinyliert ist.

12. Amplifikations-Nanoschalter-System gemäß irgendeinem der Ansprüche 1 bis 11, wobei der Bindungsarm chemisch modifiziert ist, um vor dem Digerieren durch andere lytische Enzyme, die natürlicherweise in biologischen Proben vorkommen können, geschützt zu sein.

13. Amplifikations-Nanoschalter-System gemäß irgendeinem der Ansprüche 1 bis 12, wobei die Bindungseinheit ausgewählt ist, unter:
- einer PNA-Peptid-Struktur, wobei das Peptid in der Lage ist durch einen Antikörper gebunden zu werden;
- einer PNA-Peptid-Struktur, wobei das Peptid in der Lage ist ein Antigen zu binden;
- einem Protein, das mit einem Monostreptavidin verbunden ist, wobei das Fusionsprotein in der Lage ist durch einen Antikörper gebunden zu werden;
- einem Aptamer, wobei das Aptamer in der Lage ist ein Antigen zu binden und wobei das Aptamer ferner Polymere von einer der folgenden Nukleinsäuren aufweist: Desoxyribonukleinsäure (DNA), Ribonukleinsäure (RNA), verbrückte Nukleinsäure (LNA), Peptid-Nukleinsäure (PNA), Xeno-Nukleinsäure (XNA);
- einem Spiegelmer, wobei das Spiegelmer ein Target-Molekül binden kann;
- einem Antikörper, wobei der Antikörper in der Lage ist ein Antigen oder einen anderen Antikörper zu binden;
- einem einkettigen Antikörper, wobei der einkettige Antikörper in der Lage ist ein Antigen zu binden;
- einer Antikörper-VH-Region;
- einem Affikörper;
- einem biotinylierten DNA-Strang mit mindestens 50 % Homologie zu einer Nukleinsäure-Target-Sequenz;
- einem biotinylierten RNA-Strang mit mindestens 5 % Homologie zu einer Nukleinsäure-Target-Sequenz.

14. Verfahren zur In-vitro-Detektion von Target-Analyten in einer biologischen Probe, wobei das Verfahren aufweist:
- Bereitstellen eines Amplifikations-Nanoschalter-Systems auf der Grundlage von geteilten, ortsspezifischen Spaltungsenzymen gemäß irgendeinem der Ansprüche 1 bis 13;
wobei bei Wechselwirkung eines katalytisch inaktiven Nanoschalter-Moduls (P1) mit dem anderen katalytisch inaktiven Nanoschalter-Modul (P2), induziert durch die Anwesenheit des Target-Analyten, die beiden katalytisch inaktiven Nanoschalter-Module (P1, P2) in räumliche Nähe gebracht werden, wodurch eine enzymatische Aktivität aufgrund von Enzymkomplementierung erzeugt wird, wobei die enzymatische Aktivität zur Spaltung des Effektormoleküls führt, wobei die Anwesenheit des gespaltenen Effektormoleküls die Emission eines detektierbaren und messbaren Signals auslöst, das direkt von einem Bediener oder Techniker ausgelesen oder detektiert wird und/oder das durch ein Lesegerät ausgelesen wird, dass das emittierte Signal analysiert, wobei das Ergebnis der Detektion über eine mit dem Lesegerät verbundene, grafische Schnittstelle erhalten wird, wobei das Verfahren ferner die Erhöhung der enzymatischen Aktivität des Amplifikations-Nanoschalter-Systems aufweist, wenn die Bindungsteile den Target-Analyten durch Verwendung des Verstärkermoduls binden, wodurch die Signalintensität erhöht wird.

15. Verfahren gemäß Anspruch 14, wobei die Probe eine Vollblutprobe ist.

## Revendications

1. Système de nanocommutateur d'amplification basé sur des enzymes de clivage spécifiques à un site fractionné permettant la détection *in vitro* d'analytes cibles, ledit système de nanocommutateur d'amplification comprenant :
- au moins deux modules de nanocommutateur catalytiquement inactifs (P1, P2) comprenant chacun :
- au moins un fragment d'enzyme de clivage spécifique à un site catalytiquement inactif fractionné, dans lequel ladite enzyme est une protéase ;
- au moins une fraction de liaison (BM) qui est capable de se lier à un analyte cible,
- au moins un bras de liaison configuré pour lier le au moins un fragment d'enzyme de clivage spécifique à un site catalytiquement inactif fractionné à la au moins une fraction de liaison (BM) ;
- au moins une molécule effectrice qui contient un site de reconnaissance de clivage et une molécule capteur ou rapporteur,
- un module amplificateur configuré pour augmenter une intensité de signal en augmentant l'activité enzymatique du système de nanocommutateur d'amplification lorsque les fractions de liaison se lient à l'analyte cible, ledit module amplificateur comprenant les sous-modules spécifiques suivants :
- au moins deux fragments catalytiquement inactifs de protéase liés à un ADN simple brin,
- au moins une molécule de bras de liaison liée à au moins un fragment catalytiquement inactif de protéase capable d'un auto-recuit ;
- un espaceur peptidique ou chimique ou nucléotidique contenant au moins deux sites de reconnaissance de protéase,
dans lequel ledit bras de liaison comprend, ou est constitué d'ARN, de séquences peptidiques, d'ADN, d'ANP.

2. Système de nanocommutateur d'amplification selon la revendication 1, dans lequel lors d'une interaction d'un premier module de nanocommutateur catalytiquement inactif P1 avec l'autre module de nanocommutateur catalytiquement inactif (P2) induite par la présence de l'analyte cible, les deux modules de nanocommutateur catalytiquement inactifs (P1, P2) peuvent être transportés à proximité, en générant ainsi une activité enzymatique due à une complémentation enzymatique, dans lequel l'activité enzymatique conduit au clivage de la molécule effectrice, dans lequel la présence de la molécule effectrice clivée déclenche une émission d'un signal détectable et mesurable qui est lu ou détecté directement par un opérateur ou un technicien et/ou qui est lu à travers un lecteur analysant le signal émis, moyennant quoi le résultat de la détection est obtenu à travers une interface graphique connectée audit lecteur.

3. Système de nanocommutateur d'amplification selon la revendication 1 ou 2, dans lequel ladite molécule capteur ou rapporteur est choisie parmi :
- une molécule émettant un signal qui peut être détecté par un lecteur et/ou un utilisateur analysant une variation de signal électrique en termes d'inductance, de courant, de potentiel électrique, en cas de détection conductimétrique, ampérométrique, voltammétrique, ou la présence de lumière à des longueurs d'onde spécifiques en cas de détection colorimétrique ou de détection de fluorescence/chimioluminescence, ou des phénomènes de diffusion et/ou de réfraction/diffraction de lumière, en cas de détection optique plasmonique, ou une combinaison de ceux-ci, ou
- un système extincteur/fluorophore ou un rapporteur électrochimique, ou une enzyme capable de générer un signal mesurable, ou une particule ou un composé configuré(e) pour une détection colorimétrique, en particulier des nanoparticules ou des billes qui peuvent être attachées les unes aux autres par des séquences qui peuvent être clivées par l'enzyme spécifique à un site fractionné, le clivage de la séquence induisant une désagrégation des nanoparticules ou des billes et l'induction d'un décalage de couleur, qui peut être visualisé par un lecteur optique ou par un opérateur ou un technicien.

4. Système de nanocommutateur d'amplification selon l'une quelconque des revendications 1 à 3, dans lequel ladite molécule effectrice est choisie parmi :
- une séquence peptidique qui est capable d'être clivée par une protéase complémentée, dans lequel un extincteur et un fluorophore sont conjugués à l'extrémité N-terminale et à l'extrémité C-terminale, ou vice versa, dans lequel ledit fluorophore peut être substitué par une molécule luminescente ou une enzyme et l'extincteur est substitué respectivement par un inhibiteur de ladite molécule luminescente ou un inhibiteur de ladite enzyme, ou
- une séquence peptidique qui peut être clivée par une protéase complémentée, dans lequel un rapporteur électrochimique est conjugué sur une première extrémité et l'autre extrémité est liée à une surface, en particulier une surface d'électrode, ou
- une séquence peptidique qui peut être clivée par une protéase complémentée, dans lequel un rapporteur électrochimique est conjugué sur une extrémité d'ANP et la séquence peptidique est attachée à une surface, en particulier une surface d'électrode, ou
- ladite molécule effectrice comprend deux séquences d'ANP couplées à des séquences peptidiques clivables par une protéase complémentée, qui sont suivies de séquences riches en cystéine liées par liaison thiol à une surface, notamment une surface d'électrode.

5. Système de nanocommutateur d'amplification selon la revendication 4, dans lequel ladite surface d'électrode est fonctionnalisée avec des couches de nanotube de carbone, de fullerène, ou de graphène et ledit rapporteur électrochimique est choisi parmi : le bleu de méthylène, le ferrocène, le pentaméthyl ferrocène, le ferrocène C-5, le bleu du Nil, l'anthraquinone, l'anthraquinone C-5, la thionine, le Dabcyle, le ROX, le rouge neutre, la gallocyanine, le 2,6-dichlorophénol-indophénol.

6. Système de nanocommutateur d'amplification selon l'une quelconque des revendications 1 à 5, dans lequel ladite molécule effectrice est liée à l'une quelconque des suivantes : une nanoparticule magnétique, ou une nanoparticule de point quantique, une bille d'agarose, ou une bille revêtue de streptavidine, ou une combinaison de celles-ci.

7. Système de nanocommutateur d'amplification selon l'une quelconque des revendications 1 à 6, dans lequel la cible analytique est choisie parmi : des anticorps, des protéines, des peptides, des antigènes, des composés organiques, des composés inorganiques, des composés synthétiques et des acides nucléiques, en particulier un anticorps, un affibody, un anticorps à chaîne unique, une région VH d'anticorps, un peptide, une protéine, un brin d'ADN, est un brin d'ARN, un composé inorganique, un mono-, di- ou polysaccharide, un haptène, un composé synthétique.

8. Système de nanocommutateur d'amplification selon l'une quelconque des revendications 1 à 7, dans lequel ledit système de nanocommutateur d'amplification comprend une pluralité de molécules effectrices, en particulier deux ou plus, chacune spécifique pour les différentes enzymes des fragments catalytiquement inactifs de l'enzyme du module amplificateur et pour l'enzyme de division spécifique à un site desdits modules de nanocommutateur catalytiquement inactifs.

9. Système de nanocommutateur d'amplification selon l'une quelconque des revendications 1 à 8, dans lequel ledit système de nanocommutateur d'amplification comprend des inhibiteurs chimiques configurés pour inhiber d'autres enzymes lytiques qui sont naturellement présentes dans des échantillons biologiques afin de protéger lesdits bras de liaison d'une digestion par lesdites autres enzymes lytiques.

10. Système de nanocommutateur d'amplification selon l'une quelconque des revendications 1 à 9, dans lequel ladite protéase est la protéase du virus de la gravure du tabac (TEV), en particulier dans lequel ladite protéase TEV est divisée en deux fragments de résidus 1 à 118 et de résidus 119 à 242 ou dans lequel la protéase TEV est divisée en les séquences de fragments de protéase TEV modifiées artificiellement suivantes : SEQ ID No. 1, SEQ ID No. 2, de protéase TEV artificiellement modifiée ayant la séquence SEQ ID No. 3.

11. Système de nanocommutateur d'amplification selon l'une quelconque des revendications 1 à 10, dans lequel ledit bras de liaison est choisi parmi :
- un brin d'ADN biotinylé capable de se lier au fragment catalytiquement inactif par l'intermédiaire d'une unité de streptavidine ou de monostreptavidine ;
- un brin d'ADN capable de se lier au fragment catalytiquement inactif par l'intermédiaire d'une séquence d'ADN configurée pour se lier à un marqueur d'hystidine présent sur le fragment catalytiquement inactif à l'aide d'acide nitrilotraicétique (NTA) de ligand chélatant le nickel ;
- une séquence peptidique fusionnée avec le fragment d'enzyme et avec la fraction de liaison, en créant ainsi une structure recombinante chimérique unique ;
- un brin d'ADN contigu à des fractions de liaison d'ADN et biotinyilé aux extrémités 3' ou 5'.

12. Système de nanocommutateur d'amplification selon l'une quelconque des revendications 1 à 11, dans lequel ledit bras de liaison est modifié chimiquement afin d'être protégé d'une digestion par d'autres enzymes lytiques qui peuvent être naturellement présentes dans des échantillons biologiques.

13. Système de nanocommutateur d'amplification selon l'une quelconque des revendications 1 à 12, dans lequel ladite fraction de liaison est choisie parmi :
- une structure de peptide d'ANP, dans lequel le peptide est capable d'être lié par un anticorps ;
- une structure de peptide d'ANP, dans lequel le peptide est capable d'une liaison à un antigène ;
- une protéine fusionnée avec une monostreptavidine, dans lequel la protéine de fusion est capable d'être liée par un anticorps ;
- un aptamère, dans lequel l'aptamère est capable de se lier à un antigène, l'aptamère comprenant en outre des polymères de l'un des acides nucléiques suivants : acide désoxyribonucléique (ADN), acide ribonucléique (ARN), acide nucléique verrouillé (ANL), acide nucléique peptidique (ANP), acide nucléique xéno (ANX) ;
- un Spiegelmer, dans lequel le Spiegelmer peut se lier à une molécule cible ;
- un anticorps, dans lequel ledit anticorps est capable de se lier à un antigène ou à un autre anticorps ;
- un anticorps monocaténaire, dans lequel l'anticorps monocaténaire est capable de se lier à un antigène ;
- une région VH d'anticorps ;
- un affibody ;
- un brin d'ADN biotinylé présentant une homologie d'au moins 50 % avec une séquence d'acide nucléique cible ;
- un brin d'ARN biotinylé présentant une homologie d'au moins 5 % avec une séquence d'acide nucléique cible.

14. Procédé de détection *in vitro* d'analytes cibles dans un échantillon biologique, ledit procédé comprenant les étapes consistant à :
- fournir un système de nanocommutateur d'amplification basé sur des enzymes de clivage spécifiques à un site fractionné selon l'une quelconque des revendications 1 à 13 ;
dans lequel, lors d'une interaction d'un module de nanocommutateur catalytiquement inactif (P1) avec l'autre module de nanocommutateur catalytiquement inactif (P2) induite par la présence de l'analyte cible, les deux modules de nanocommutateur catalytiquement inactifs (P1, P2) sont transportés à proximité, en générant ainsi une activité enzymatique due à une complémentation enzymatique, dans lequel l'activité enzymatique conduit au clivage de la molécule effectrice, dans lequel la présence de la molécule effectrice clivée déclenche une émission d'un signal détectable et mesurable qui est lu ou détecté directement par un opérateur ou un technicien et/ou qui est lu à travers un lecteur analysant le signal émis, moyennant quoi le résultat de la détection est obtenu à travers une interface graphique connectée audit lecteur, ledit procédé comprenant en outre une augmentation de l'activité enzymatique du système de nanocommutateur d'amplification lorsque les fractions de liaison se lient à l'analyte cible en utilisant ledit module amplificateur, en augmentant ainsi l'intensité de signal.

15. Procédé selon la revendication 14, dans lequel ledit échantillon est un échantillon de sang total.
